# EUROPEAN PATENT APPLICATION

(11) **EP 3 845 242 A1**
(43) Date of publication of application: **07.07.2021**
(21) Application number: 19854211.0
(22) Date of filing: 20.08.2019
(51) Int. Cl.: A61K 39/39, A61K 39/35, A61P 37/06, A61P 37/08, A61P 43/00

(54) **IMMUNE TOLERANCE-INDUCING AGENT AND THERAPEUTIC OR PROPHYLACTIC AGENT FOR ALLERGIC DISORDER**

(30) Priority: 30.08.2018 JP 2018161929
(71) Applicant: Tokushima University, Tokushima-shi, Tokushima 770-8501 (JP)
(72) Inventor: KIDO, Hiroshi, Tokushima-shi, Tokushima 770-8501 (JP); TAKAHASHI, Etsuhisa, Tokushima-shi, Tokushima 770-8501 (JP); KIMOTO, Takashi, Tokushima-shi, Tokushima 770-8501 (JP); SAKAI, Satoko, Tokushima-shi, Tokushima 770-8501 (JP)
(74) Representative: Hamer, Christopher K.
(86) International application number: PCT/JP2019/032352
(87) International publication number: WO 2020/045155

(57) **Abstract**

An object of the present invention is to provide an allergen vaccine for treatment or prevention of an allergic disease, while reducing the risk of developing an immediate type allergy including anaphylaxis. The present inventors have found that (1) oral inoculation of a composition comprising a particular antigen (allergen) and SF10 into subjects having allergic diseases can suppress the development of an immediate type allergy caused by sensitization with the antigen, and (2) oral inoculation of a composition comprising a particular antigen (allergen) and SF10 into subjects not having allergic diseases can inhibit establishment of sensitization to the antigen, and have completed the present invention.

## Description

### Technical Field

The present invention relates to an immune tolerance-inducing agent or an agent for treatment or prevention of allergic diseases for oral administration, comprising a pulmonary surfactant-derived synthetic mucosal adjuvant SF10 (sometimes described in Patent and Non-patent Documents as SF-10) and an antigen. The immune tolerance-inducing agent or an agent for treatment or prevention of allergic diseases of the present invention can be used in an oral immunotherapy for treatment or prevention of allergic diseases, in particular food allergies.

### Background Art

An allergy is an immune response that occurs by exposure to a causative substance (allergen), and brings disadvantages to a living body. Since humans maintain life by digesting and absorbing xenobiotic organisms as foods, they are in a state where undigested foreign matters which are not digested enough are constantly taking up into the body. It is assumed, however, that immune tolerance (immunotolerance) normally works to prevent excessive biological defensive reactions (allergic reactions) against such undigested foreign substances. However, currently, 5 to 10% of infants in the birth population suffer from some kind of allergic diseases such as food allergy by the first year of life. Many of them are naturally cured by the age of 5 to 6 years old, but when not cured, the allergic diseases may progress to severe allergic symptoms (allergic march) accompanied by atopic dermatitis and asthma, or the like. Meanwhile, allergies may be developed by allergens in the environment (pollen, mold, house dust, or the like) with increasing age, followed by the breakdown of immune tolerance so far. In developed countries, such allergies continue to increase year by year, and it is said that about 30% of the entire population suffers from some kind of allergic diseases, thus countermeasure to them are strongly desired.

In the past, there have been no effective treatment for allergic diseases, and symptomatic treatments aimed at reducing allergic symptoms are generally used. However, in recent years, allergen immunotherapy (also called desensitization therapy) has been developed as a curative treatment for allergic disease, and attracting attention. Allergen immunotherapy is a therapy of administering an allergen (antigen) to a patient under the control of a physician in an attempt to induce immune tolerance. As allergen immunotherapies, oral immunotherapy for food allergy (Patent Documents 1 and 2), sublingual immunotherapy for pollen or mite allergy (Patent Document 3), and transdermal immunotherapy (Patent Document 4) are known.

Allergen immunotherapy starts with a low allergen dose and the dose is gradually increases to safely conduct immunotherapy, but there is such a risk that administering allergen at the dose above a threshold will cause serious immediate allergy symptoms. Thus, standardized allergen vaccines are used in allergen immunotherapy. Allergen vaccines reduce the risk of eliciting immediate allergic reactions while maintaining allergenicity capable of inducing immune tolerance, by processing natural allergens (by extraction of allergen components, heat treatment, chemical modification, formulation to the sustained release form, or the like). Examples of allergen vaccines disclosed include an egg allergy therapeutic composition that contains a heat-denatured and powdered egg (Patent Documents 5 and 6) and a cow's milk allergy therapeutic composition that contains modified β-lactoglobulin (Patent Document 7). Also disclosed are a composition for treating cedar hay using a fusion protein of Cryj1 and Cryj2, which are major allergen proteins of cedar pollen (Patent Document 8), and a composition for treating cedar hay using another cedar pollen allergen protein (Patent Document 9).

However, it is known that even with such an allergen vaccine, it is difficult to completely prevent the development of severe allergies through desensitization therapy (Non-patent Documents 1 to 3). In particular, the risk of desensitization therapy is even higher in food allergies because patients include infants and young children. Indeed, several cases have been reported in Japan in which oral immunotherapy has caused severe allergic symptoms (anaphylaxis) including cardiopulmonary arrest. From the above, there is a strong demand for development of a safer allergen vaccine (an immune tolerance-inducing agent) for oral immunotherapy.

Meanwhile, "pulmonary surfactant" secreted from human alveolar type II cells is known as a nostrum for neonatal respiratory distress syndrome. The present inventors have paid attention in advance to the availability of "pulmonary surfactant" as an adjuvant, and have developed a synthetic pulmonary surfactant (SSF) capable of mass production (Patent Documents 10 to 12). In addition, the present inventors have found that addition of a thickener carboxyvinyl polymer (CVP) can extend time of antigen delivery by SSF, and have developed a synthetic mucosal adjuvant SF10 containing SSF and CVP (Patent Document 13, Non-patent Document 4, and Non-patent Document 5).

The present inventors have also revealed that the use of SF10 as an adjuvant of influenza vaccine for nasal inoculation results in balanced induction of Th1 and Th2 immune systems and an increase of influenza antigen-specific IgA not only in blood, but also in nasal and bronchoalveolar lavage fluids, and no adverse effects including inflammatory reactions (Non-patent Document 4, Non-patent Document 5, and Non-patent Document 6). However, oral administration of SF10 has not been attempted at all, and its effects are completely unknown.

### Prior Art Documents

### Patent Documents

Patent Document 1: Japanese Patent No. 4843792
Patent Document 2: Japanese Patent No. 5028627
Patent Document 3: Japanese Patent No. 5473899
Patent Document 4: Japanese Patent No. 5804278
Patent Document 5: Japanese unexamined Patent Application Publication No. 2015-105234
Patent Document 6: Japanese unexamined Patent Application Publication No. 2015-104331
Patent Document 7: Japanese unexamined Patent Application Publication (Translation of PCT Application) No. 2011-525891
Patent Document 8: WO 2007/080977
Patent Document 9: Japanese unexamined Patent Application Publication No. 2008-141993
Patent Document 10: WO 2005/097182
Patent Document 11: WO 2007/018152
Patent Document 12: WO 2009/123119
Patent Document 13: WO 2011/108521

### Non-patent Documents

Non-patent Document 1: Ventura MT et al. Immunopharmacol Immunotoxicol. 30: 153-61, 2008
Non-patent Document 2: Rezvani M et al. Immunol Allergy Clin North Am. 27: 295-307,2007
Non-patent Document 3: Bernstein DI. Allergy. 63:374, 2008
Non-patent Document 4: Kimoto T et al. Influenza and Other Resp. Viruses 7(6): 1218-1226, 2013.
Non-patent Document 5: Mizuno D et al. Vaccine 34(16) : 1881-1888, 2016.
Non-patent Document 6: Kim H, et al. PLOS ONE 13(1):e0191133, 2018.

### Summary of the Invention

### Object to be Solved by the Invention

An object of the present invention is to develop an allergen immune tolerance-inducing agent (sometimes referred to as an "immune tolerance-inducing vaccine") that uses SF10 as an adjuvant, and to provide an immune tolerance-inducing agent for treatment or prevention of allergic diseases or an agent for treatment or prevention of allergic diseases while reducing the risk of developing immediate allergy including anaphylaxis.

### Means to Solve the Object

Human pulmonary surfactant is produced in large quantities from alveolar type II cells after 34 weeks of gestation, and accumulates in amniotic fluids by forming a fetal fat-pulmonary surfactant complex. It is known that the fetus constantly swallows this fetal fat-pulmonary surfactant complex, and pulmonary surfactant is selectively absorbed in the upper gastrointestinal mucosa and promotes the maturation of the intestinal mucosa at the site of absorption (Nishijima K, et al. Am J Physiol Lung Cell Mol Physiol. 2012; 303: L208-L214.). From such reports, the present inventors have considered that oral administration of a complex comprising an allergen (antigen) and SF10 (hereinafter referred to as "allergen-SF10" or "antigen-SF10") would lead selective absorption of SSF (and an allergen combined therewith) in the upper gastrointestinal mucosa to cause an efficient intestinal immune response, and would be able to induce an immune response with a lower amount of allergen (i.e., allergen in an amount below the reaction threshold) compared to the administration of allergen alone.

The present inventors have then prepared complexes containing various allergens and SF10 as allergen immune tolerance-inducing agents and investigated their effects using an allergen transdermally sensitized mouse model (body weight of about 20 g). As a result, it has been revealed that i) administration of a complex of OVA with SF10 (OVM-SF10) as a therapeutic allergen immune tolerance-inducing agent to an ovomucoid (OVA) transdermally sensitized allergic mouse nearly completely suppresses the development of anaphylaxis by an OVA oral administration load test; ii) the vaccine effect of OVM-SF10 administered as a therapeutic allergen immune tolerance-inducing agent is highest when the OVM content is 0.01 µg per mouse (i.e., an amount that does not affect the mouse immune response by antigen alone); iii) oral inoculation of a complex of casein with SF10 (casein-SF10) beforehand as a prophylactic allergen immune tolerance-inducing agent into healthy (non-allergen sensitized) mouse does not develop a casein allergy, even with a subsequent casein transdermal sensitization.

The present inventors have found unexpected results that oral inoculation of an antigen (allergen)-SF10 complex induces immune tolerance to the antigen (allergen) in situations where nasal inoculation of an antigen-SF10 complex is known to induce an immune response to the antigen, and have completed the present invention.

That is, the present invention relates to (1) an immune tolerance-inducing agent for oral administration, comprising a pulmonary surfactant-derived synthetic mucosal adjuvant SF10 and an antigen; (2) the immune tolerance-inducing agent according to "1", wherein the antigen is one or more antigens selected from ovomucoid, ovalbumin, and casein; (3) an agent for treatment or prevention of an allergic disease, comprising an immune tolerance-inducing agent for oral administration as an active ingredient, wherein the immune tolerance-inducing agent comprises a pulmonary surfactant-derived synthetic mucosal adjuvant SF10 and an antigen; (4) the agent for treatment or prevention of an allergic disease according to "3", wherein the treatment or prevention is an oral immunotherapy; (5) the agent for treatment or prevention of an allergic disease according to "3" or "4", wherein the allergic disease is a food allergy; and (6) the agent for treatment or prevention of an allergic disease according to "5", wherein the food allergy is a cow's milk allergy or an egg allergy.

### Effects of the Invention

Oral administration of the immune tolerance-inducing agent of the present invention can induce and establish immune tolerance even with the antigen in an amount that is lower than the anaphylaxis reaction threshold, due to the adjuvant effect of SF10. Thus, when the immune tolerance-inducing agent of the present invention is used as an allergen vaccine, an oral immunotherapy can be carried out safely and efficiently in a subject having allergic diseases. Prevention of allergic diseases can also be carried out by administering the immune tolerance-inducing agent of the present invention to subjects that do not have allergic diseases.

### Brief Description of Drawings

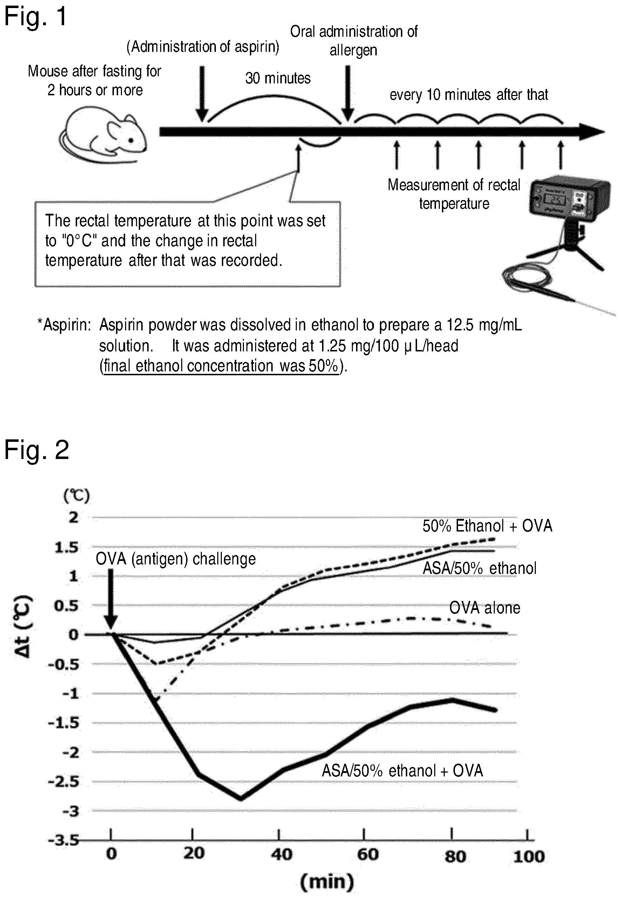

[Figure 1] Figure 1 is a diagram showing a protocol of an anaphylaxis-eliciting experiment using the allergic mouse model of the present invention. Oral dosing of aspirin to the mice 30 minutes before anaphylaxis elicitation amplifies anaphylaxis elicitation by oral allergen (antigen) challenge, and enables to reliably evaluate a suppression effect of an immune tolerance-inducing agent on anaphylaxis reaction.
[Figure 2] Figure 2 is a diagram showing the results of an anaphylaxis-eliciting experiment using the transdermally sensitized allergic mouse model (body weight of about 20 g) of the present invention. The transdermally sensitized allergic mouse model is used for the experiment after becoming an anaphylaxis-eliciting condition one to two weeks after transdermal sensitization with allergen (OVA) five times a week for two weeks. The vertical axis of the graph shows the change in mouse rectal temperature, and the horizontal axis shows the time after oral challenge with allergen (OVA). ASA: aspirin (dissolved in 50% ethanol) is orally administered 30 minutes before the OVA challenge. OVA: ovalbumin.

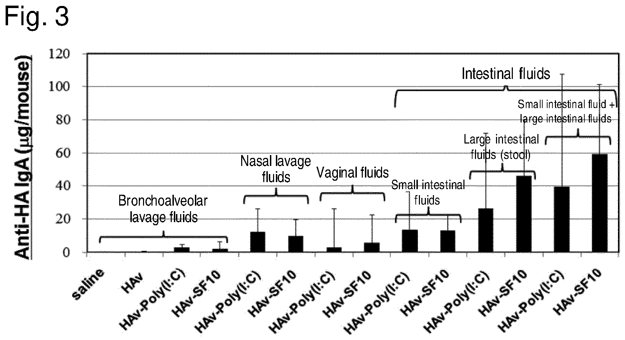

[Figure 3] Figure 3 is a diagram showing a promoting effect of induction of HAv-specific IgA antibodies contained in each immune organ of mouse (body weight of about 20 g) two weeks after nasal inoculation of a complex of influenza vaccine antigen (HAv) with SF10. The vertical axis of the graph shows the amount of HAv-specific IgA antibody contained in bronchoalveolar lavage fluids, nasal lavage fluids, vaginal fluids, and intestinal fluids (small intestinal fluids, large intestinal fluids (stool), small intestinal fluids + large intestinal fluids) per mouse. In addition, "saline" on the horizontal axis of the graph indicates the administration group of saline alone that is used as a control solvent, "HAv" indicates the HAv alone administration group, "HAv-Poly (I : C)" indicates the administration group of a combination of HAv and Poly (I : C), and "HAv-SF10" indicates the administration group of a complex of HAv with SF10, respectively.

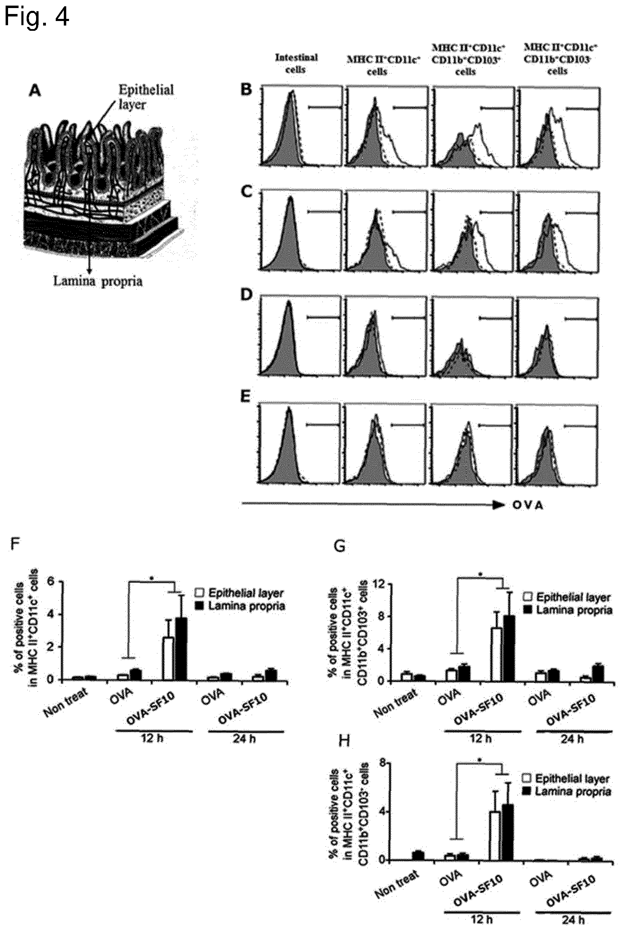

[Figure 4] Figure 4A shows a schematic diagram of an intestinal structure, and Figures 4B-E show the results of histogram analysis of uptake of the complex of the present invention (a complex of fluorescently labeled OVA with SF10) into dendritic cells. In these figures, dendritic cells are shown as a population of cells to be stained with MHCII⁺CD11c⁺ antibodies. The dendritic cell population is further divided into two types: CD11b positive, CD103 positive cells (MHCII⁺CD11c⁺CD11b⁺CD103⁺) ; and CD11b positive, CD103 negative cells (MHCII⁺CD11c⁺CD11b⁺CD103⁻). The figures show the results of dividing the cells in a total of the three types of dendritic cell populations analyzed by dendritic cell marker antibodies. The indications of gray color show the results of the untreated group, the indications of dotted line show the results of the fluorescently labeled OVA alone oral administration group, and the indications of solid line show the results of the fluorescently labeled OVA-SF10 oral administration group. Figures 4F-H show the percentage (%) of dendritic cells taking up the fluorescently labeled OVA in the three types of dendritic cell populations. By complexing SF10 adjuvant with the fluorescently labeled OVA, a significant increase of the fluorescently labeled OVA taken up into the dendritic cells was observed in any dendritic cells 12 hours after oral administration compared to that in the fluorescently labeled OVA alone administration group. Twenty-four hours later, this uptake enhancement effect of SF10 disappeared.

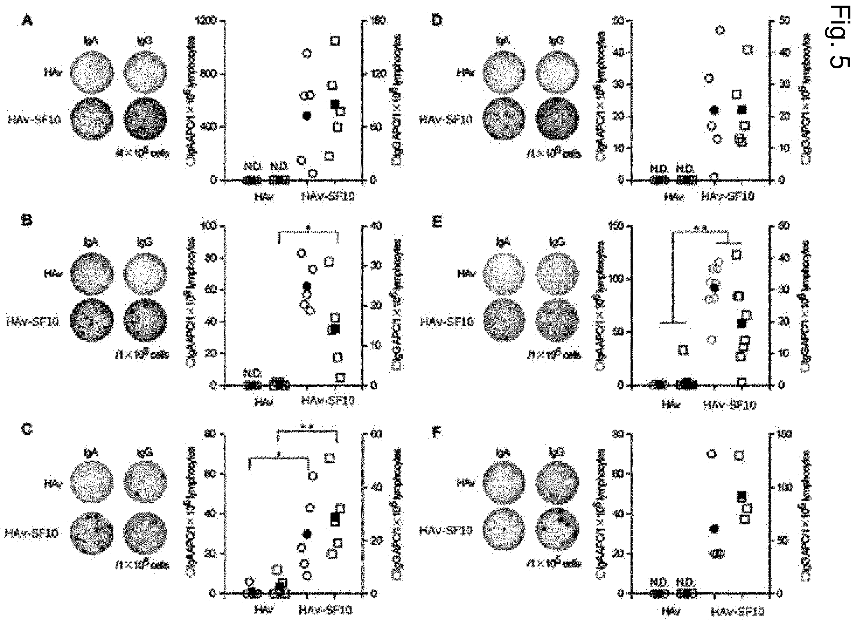

[Figure 5] Figure 5 is a diagram showing an increase in the numbers of HAv-specific IgA and IgG-producing cell colony spots in systemic lymphoid tissues of mouse (body weight of about 20 g) after oral immunization with a complex of influenza antigen (HAv) with SF10 (HAv-SF10). In Figure 5, A shows the result of HAv-specific IgA and IgG-producing cell population (colony) numbers of lymphocytes in the lung lymph nodes, B in spleen, C in cervical lymph nodes, D in mediastinal chest lymph nodes, E in Peyer's patch, and F in gastric lymph nodes. Compared to the HAv alone oral immunization group, the HAv-SF10 oral immunization group showed a significant increase in the numbers of IgA and IgG-producing colonies. In the graph on the right of each figure, "filled circle" indicates the mean value of HAv-specific IgA-secreting cell colony numbers, and "filled square" indicates the mean value of HAv-specific IgG-secreting cell colony numbers.

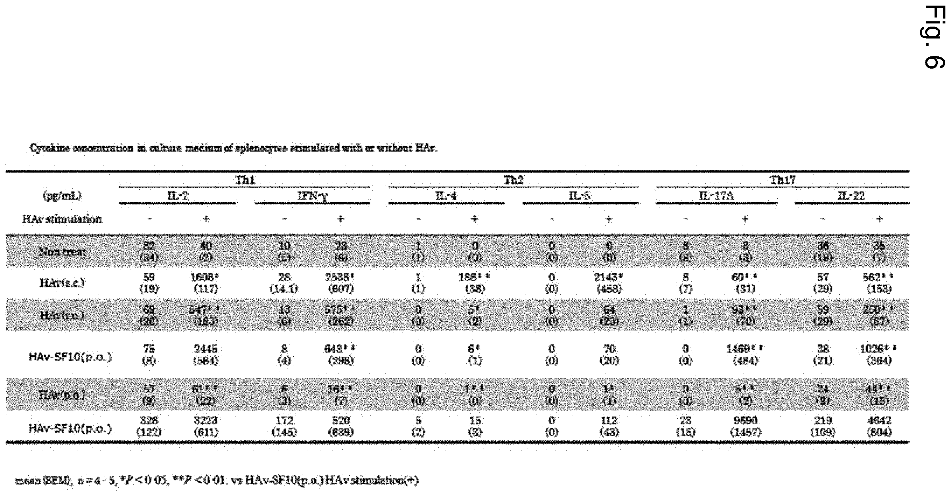

[Figure 6] Figure 6 shows Th1, Th2, Th17 cytokine secretion amount responses with or without HAv stimulation under culture conditions of mouse splenocytes collected after subcutaneous, nasal, or oral immunization of mouse (body weight of about 20 g) with a complex of influenza antigen (HAv) with SF10. In the figure, "s.c." indicates the subcutaneous inoculation group, "i.n." indicates the nasal inoculation group, and "p.o." indicates the oral administration group, respectively. IL-1 and IFN-γ were measured as Th1 cytokines, IL-4 and IL-5 as Th2 cytokines, and IL-17A and IL-22 as Th17 cytokines. While HAv (s.c.) immunization predominantly induced Th1 and Th2 cytokines, HAv-SF10 (p.o.) immunization showed the most potent induction of Th17 and Th1 cytokines and relatively mild induction of Th2 cytokines.

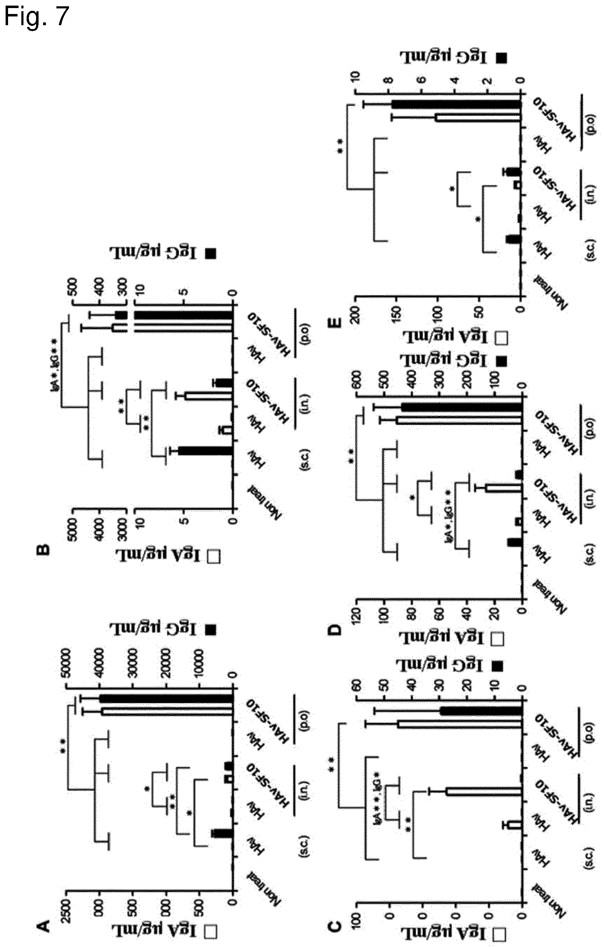

[Figure 7] Figure 7 is a diagram showing HAv-specific IgA and IgG production enhancement effects in each organ of mouse (body weight of about 20 g) after subcutaneous, nasal, or oral immunization of a complex of influenza antigen (HAv) with SF10. In Figure 7, A shows the results of blood, B bronchoalveolar lavage fluids, C nasal lavage fluids, D stomach extracts, and E stool extracts, respectively. The vertical axis of the graph shows HAv-specific antibody concentration in the sample (white column: IgA, black column: IgG). The horizontal axis of the graph shows groups of different inoculation routes, and "s.c." indicates the subcutaneous inoculation group, "i.n." indicates the nasal inoculation group, and "p.o." indicates the oral administration group, respectively.

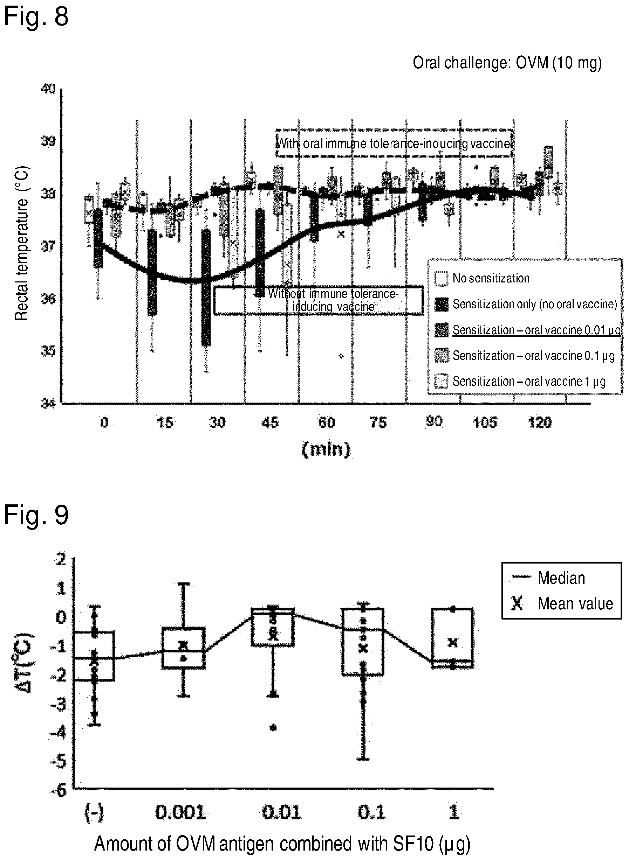

[Figure 8] Figure 8 is a diagram showing the results of an anaphylaxis-eliciting test by an oral allergen challenge following oral immunization of the transdermally sensitized allergic mouse model (body weight of about 20 g) of the present invention with a complex of OVM with SF10 (OVM-SF10) as an immune tolerance-inducing vaccine. The vertical axis of the graph shows the mouse rectal temperature and the horizontal axis shows the time after OVM oral administration (challenge test), respectively. Also in the figure, "vaccine" indicates the immune tolerance-inducing vaccine of the present invention (a complex of OVM with SF10), and "sensitization" indicates induction into an anaphylaxis-eliciting condition by transdermal sensitization with OVM. That is, in this figure, "no sensitization" refers to the group in which an OVM oral challenge was carried out on healthy mouse, "sensitization only (no vaccine)" refers to the group in which an OVM oral challenge was carried out on allergic mouse model that is in an anaphylaxis-eliciting condition by transdermal sensitization with OVM, and "sensitization + vaccine" refers to the group in which an OVM oral challenge was carried out on the above sensitized allergic mouse model after oral immunization of the immune tolerance-inducing vaccine (the complex of the present invention), respectively.
[Figure 9] Figure 9 is a diagram showing the results of examining how OVM content affects the immune tolerance-inducing vaccine effect by a complex of OVM with SF10 (OVM-SF10). The vertical axis of the graph shows the change in mouse rectal temperature after the OVM challenge test of mouse with body weight of about 20 g, as the median and the mean value (calculated using the temperature before the load test as a reference value). The horizontal axis of the graph indicates the amount of OVM antigen contained in the immune tolerance-inducing vaccine, and " (-)" indicates the vaccine non-administration group, "0.001" indicates the OVM (0.001 µg)-SF10 administration group, "0.01" indicates the OVM (0.01 µg)-SF10 administration group, "0.1" indicates the OVM (0.1 µg)-SF10 administration group, and "1" indicates the OVM (1 µg)-SF10 administration group, respectively.

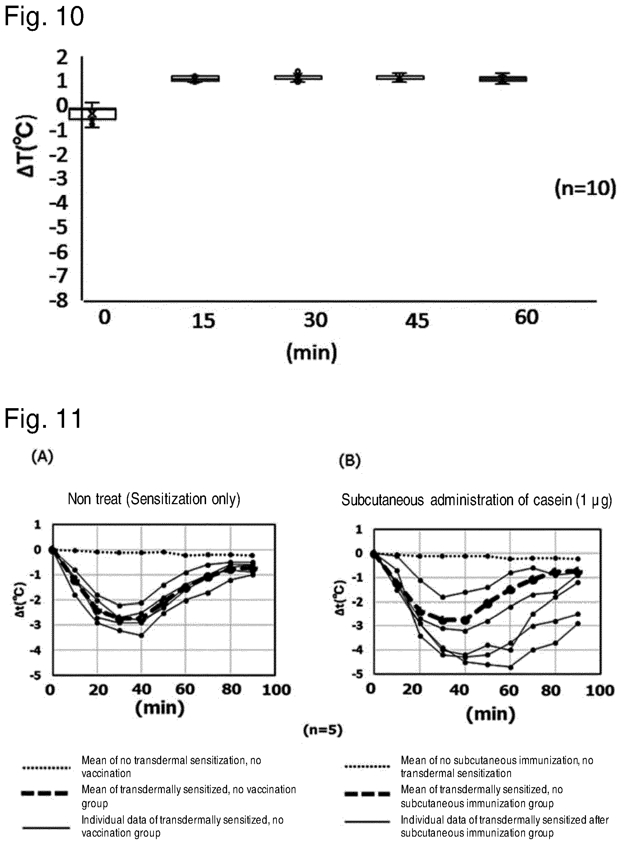

[Figure 10] Figure 10 is a diagram showing rectal temperature change due to anaphylaxis reaction when 0.01 µg of OVM alone was administered orally to the OVM transdermally sensitized allergic mouse model of the present invention (body weight of about 20 g, n = 10). The vertical axis of the graph shows the change in rectal temperature by boxplot graph, and the horizontal axis shows the time after OVM alone administration, respectively. It should be noted that 0.01 µg of OVM is the OVM content contained in the OVM-SF10 immune tolerance-inducing vaccine that showed the best immune tolerance-inducing effect.
[Figure 11] Figure 11 is a diagram showing the results of examining the prophylactic effect of transdermal allergen immunotherapy by subcutaneous injection of casein. Figure 11(A) shows the individual data and the mean value of an anaphylaxis-eliciting test performed by casein oral challenge using casein transdermally sensitized allergic mouse model (body weight of about 20 g, n = 5). Figure 11(B) shows the individual data and the mean value thereof obtained by performing subcutaneous injection of casein to mouse (body weight of about 20 g) beforehand as a prophylactic transdermal allergen immunotherapy, and after two weeks, performing transdermal sensitization with casein, and then two weeks after completion of the sensitization, performing anaphylaxis-eliciting test by oral casein challenge (n = 5). The "mean of no subcutaneous immunization, no vaccination" in Figures11(A) and (B) shows the results (thin dotted line) of an oral casein challenge in healthy mouse (no prophylactic subcutaneous immunization of casein, no transdermal sensitization with casein). The "mean of transdermally sensitized after subcutaneous immunization group" in (B) shows the mean value (thick dotted line, n = 5) of the results of performing prophylactic subcutaneous immunization beforehand with casein twice at two-week interval, and then performing transdermal sensitization with casein, and then two weeks after completion of the sensitization, performing oral casein challenge test. The individual data thereof is shown by thin solid line. The vertical axis of the graph shows the change in mouse rectal temperature due to the anaphylaxis reaction after the casein challenge test (calculated using the temperature before the challenge test as a reference value), and the horizontal axis shows the time after the challenge test.

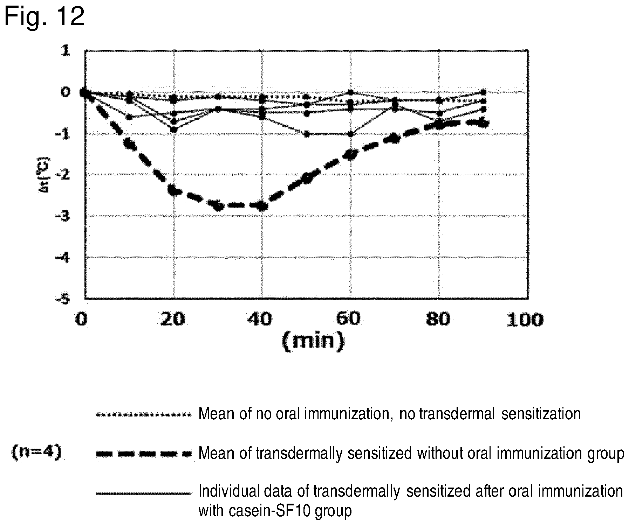

[Figure 12] Figure 12 is a diagram showing the results of examining the prophylactic effect on casein allergy by prophylactic oral administration of a complex of casein with SF10 (casein-SF10 immune tolerance-inducing vaccine).
Figure 12 shows the results of performing an oral casein challenge test using mouse that had oral inoculation of a casein-SF10 immune tolerance-inducing vaccine twice (into mouse with body weight of about 20 g, the first time and three days later) and two weeks after final immunization, transdermally sensitization with casein (n = 4). The "mean of no oral immunization, no transdermal sensitization" in Figure 12 shows the mean of the results of challenge test in healthy mouse (no prophylactic oral immunization of casein, no transdermal sensitization with casein) by thin dotted lines. The "mean of transdermally sensitized without oral immunization group" shows the mean value of the results of performing oral casein challenge after casein transdermal sensitization to mouse without prophylactic oral inoculation of casein-SF10 (mean value of 4 cases, respectively) by thick dotted lines. Furthermore, "individual data" obtained by performing casein transdermal sensitization to mouse that had prophylactic oral inoculation of casein-SF10 immune tolerance-inducing vaccine and then performing oral casein challenge are shown by thin solid lines. The vertical axis of the graph shows the change in mouse rectal temperature after the anaphylaxis-eliciting challenge test (calculated using the temperature before the load test as a reference value), and the horizontal axis shows the time after the load test.

### Mode of Carrying Out the Invention

The "immune tolerance-inducing agent" of the present invention (herein sometimes referred to as an "immune tolerance-inducing vaccine") comprises SF10 and an antigen, and is not particularly limited as long as its oral administration may induce immune tolerance to the antigen. The "induction of immune tolerance" means: (i) suppressing the onset, (ii) alleviating a symptom, (iii) delaying progression, or (iv) promoting recovery of an immediate allergic reaction including anaphylaxis in a subject having allergic diseases; and in addition to (i)-(iv) above, further (v) inhibiting or suppressing establishment of sensitization to an allergen (antigen) in a subject not having allergic diseases. In the present specification, the "immune tolerance-inducing agent" may be referred to as a "therapeutic allergen immune tolerance-inducing agent" when used in the treatment of allergic diseases, and may be referred to as a "prophylactic allergen immune tolerance-inducing agent" when used in the prevention of the onset of allergic diseases.

The "SF10" used in the present invention means an adjuvant containing: (a) a synthetic pulmonary surfactant (SSF) composed of a synthetic peptide consisting of an amino acid sequence of KnLm (provided that n is 4 to 8 and m is 11 to 20) and lipids; and (b) a carboxyvinyl polymer (CVP). Preferred examples of the "lipids" include phosphatidylcholine, dipalmitoylphosphatidylcholine, phosphatidylserine, phosphatidylglycerol, phosphatidylinositol, phosphatidylethanolamine, phosphatidic acid, lauric acid, myristic acid, palmitic acid, stearic acid, and oleic acid, and among them particularly preferred is a combination of dipalmitoylphosphatidylcholine, phosphatidylglycerol, and palmitic acid.

The "antigen" used in the present invention means an allergen that causes an allergic disease. The "antigen" may be any allergen derived from a food, a plant, an animal, or a fungus. Specific examples of the "food-derived allergen" can include allergens derived from egg, milk, wheat, soybean, buck wheat, peanut, beef, chicken, pork, sesame, gelatin, yam, matsutake, salmon roe, fruit (e.g., orange, kiwi fruit, peach, apple, and banana), crustaceans (e.g., shrimp and crab), fish (e.g., salmon and mackerel), shellfish (e.g., abalone and squid), and seed species (e.g., cashew nuts and walnuts), and among them, the food-derived allergen is preferably an allergen derived from egg or milk, and particularly preferably ovomucoid (OVM), ovalbumin (OVA), or casein.

Examples of the above "plant-derived allergen" can include allergens derived from pollen of trees (e.g., pollen of red cedar, acacia, white alder, white ash, American beech, white birch, box elder, mountain cedar, eastern cottonwood, cypress, American elm, Chinese elm, Pinaceae, sweetgum, eucalyptus tree, hackberry, hickory, American basswood, sugar maple, mesquite, mulberry, oak, olive, pecan, pepper, pine, privet, Russian olive, American sycamore, ailanthus, black walnut, black willow, and the like); and such as pollen of grass and vegetables (pollen of cotton, Bermuda, Kentucky bluegrass, brome, corn, meadow fescue, Johnsongrass, oat, orchard, redtop, perennial rye, rice, sweet vernal, timothy, carelessweed, chenopodium, cocklebur, yellow dock, goldenrod, kochia, lambs quarters, calendula, nettle, rough pigweed, English plantain, tall ragweed, short ragweed, Western ragweed, Russian thistle, common sagebrush, common broom, sheep sorrel, and the like). Examples of the "animal-derived allergen" can include allergens derived from mites (tropical rat mite, dust mite, cheyletid mite, flour mite, tick, itch mite and the like), mammals (e.g., dog, cat, and mouse), and insects (e.g., bee, hornet, ant, and cockroach), and the like. Examples of the "fungal-derived allergen" can include allergens derived from *Alternaria, Aspergillus, Botulinus, Candida, Cephalosporium, Curvularia sp., Epicoccum nigrum, Epidermophyton, Fusarium sp., Helminthosporium sp.,* chain of *Cladosporium sp., mucor, Penicillium, Phoma sp., Pluraria Plurance, Rhizopus,* and the like.

Furthermore, the "antigen" used in the present invention may be a "natural allergen component" contained in a food, a plant, an animal, or a fungus, or may be a "specific allergen molecule" consisting of a portion of such a natural allergen component. In addition, the "specific allergen molecule" may be isolated and purified from a natural allergen component, or may be synthesized artificially using genetic recombination technology or peptide synthesis technology. Furthermore, the "natural allergen component" or "specific allergen molecule" may be subjected to a denaturation treatment (e.g., thermal denaturation or chemical modification) so that its allergy-inducing ability is reduced or increased.

The immune tolerance-inducing agent of the present invention may contain one of the above antigens, or may contain two or more of the above antigens in combination. The mass ratio (antigen/phospholipids) of the above antigen to the above lipids (phospholipids contained in SSF) in the immune tolerance-inducing agent of the present invention is preferably 0.01 to 100, more preferably 0.05 to 10, even more preferably 0.07 to 2, and particularly preferably 0.1 to 1.

Since the immune tolerance-inducing agent of the present invention can be used for treatment or prevention of allergic diseases, the present invention also relates to an agent for treatment or prevention of allergic diseases, comprising the immune tolerance-inducing agent of the present invention as an active ingredient. The "allergic diseases" is not particularly limited as long as it is allergic diseases caused by exposure to the above antigens, and preferred specific examples include food allergies, allergic rhinitis (such as hay fever), atopic dermatitis, allergic conjunctivitis, allergic gastroenteritis, bronchial asthma, asthma, and urticaria, and among them, the "allergic disease" is preferably a food allergy to be a target of oral immunotherapy, and more preferably an egg allergy or a cow's milk allergy. In the "treatment of allergic diseases", orally administering the immune tolerance-inducing agent of the present invention once, preferably twice, more preferably three times, further preferably four times, particularly preferably five or more times, to a subject having the above allergic disease (human, or non-human animal such as pets or domestic animal) induces immune tolerance to the antigen in the subject, and ameliorates or radically cures the symptoms. Furthermore, in the "prevention of allergic diseases", orally administering the "immune tolerance-inducing agent" of the present invention once, preferably twice, more preferably three times, further preferably four times, particularly preferably five or more times, to a subject not having the above allergic disease (human or non-human animal such as pets or domestic animal) can induce immune tolerance to the antigen in the subject, and prevent the development of the allergic disease caused by a subsequent exposure to the antigen.

The "content of antigen" in the immune tolerance-inducing agent or the agent for treatment or prevention of allergic diseases of the above present invention is preferably 0.001 to 1000 µg/Kg body weight, more preferably 0.01 to 100 µg/Kg body weight, further preferably 0.05 to 50 µg/Kg body weight, more preferably 0.05 to 5 µg/Kg body weight, and particularly preferably 0.05 to 0.5 µg/Kg body weight per inoculation amount. Among these, in a case where a therapeutic immune tolerance-inducing agent is used for a subject having a high risk of developing anaphylaxis due to allergen sensitization, when OVM or OVA is used as the antigen, it is preferable that the therapeutic immune tolerance-inducing agent contains OVM or OVA at a low concentration (e.g., 50 µg or less/Kg body weight, preferably 5 µg or less/Kg body weight, more preferably 0.5 µg or less/Kg body weight per inoculation amount). Although the inoculation amount varies depending on the type of allergen, it is preferable that the amount is 0.1 times or less, more preferably 0.02 times or less of the anaphylaxis-eliciting amount of the antigen revealed in an anaphylaxis-eliciting test performed beforehand, and should fall within the above range. Even a trace amount of antigen that does not fall within the above range can amplify and achieve the immune tolerance-inducing effect by increasing the number of administrations. The immune tolerance-inducing agents or agents for treatment or prevention of allergic diseases of the present invention may contain weak alkali buffers (for example, a carbonate buffer or a phosphate buffer) for preventing digestion of antigen by gastric fluids or inactivation in an acidic environment, and may further be encapsulated in a capsule or contain a jelly-like protective agents against digestive enzymes, or the like.

The immune tolerance-inducing agents or agents for treatment or prevention of allergic diseases of the present invention can also be used concurrently with another allergen vaccines (such as allergen extracts) for allergen immunotherapy, or can also be used before or after a use of another allergen vaccines for allergen immunotherapy. The immune tolerance-inducing agents of the present invention can also be used in combination with known agents for treating allergic diseases, such as tranilast, clemastine fumarate, cyproheptadine hydrochloride, diphenhydramine, methodiramine, clemizole, or methoxyphenamine. In addition, to the immune tolerance-inducing agents or agents for treatment or prevention of allergic diseases of the present invention, pharmacologically acceptable carriers, excipients, binders, fragrances, flavor modifiers, sweeteners, colorants, isotonic agents, antiseptic agents, antioxidants, solubilizers, dissolution aids, suspending agents, fillers, pH modifiers, stabilizers, absorption accelerators, release rate control agents, plasticizers, crosslinkers, tackifiers, or surfactants can optionally be added.

Furthermore, the present invention relates to a treatment or prevention for allergic diseases; a method for inducing immune tolerance, comprising a step of orally inoculating a subject in need thereof with a complex comprising SF10 and an antigen (immune tolerance-inducing vaccines); a complex comprising SF10 and an antigen for use as an immune tolerance-inducing agent; and a use of a complex comprising SF10 and an antigen in manufacture of a medicament for inducing immune tolerance.

Hereinafter the present invention will be described in more detail by Examples, but the technical scope of the present invention is not limited to these examples.

### Examples

### Example 1

### [Preparation of food allergic mouse model]

Balb/c mice (6-7 weeks old, female) were subjected to hair removal on the back of the head with hair clippers, then, 100 µL of aqueous SDS solution (4%) was uniformly applied to damage their skin barrier function, and after 10 minutes, 100 µL of an aqueous solution of ovomucoid (OVM) (manufactured by NACALAI TESQUE, INC.) (10 mg/mL), 100 µL of an aqueous solution of ovalbumin (OVA) (manufactured by Sigma-Aldrich) (10 mg/mL), or 100 µL of an aqueous solution of casein (manufactured by Sigma-Aldrich) (10 mg/mL) was uniformly applied. Such allergen applications were carried out a total of 10 times (for 2 weeks with a frequency of 5 times/week) to generate a transdermally sensitized food allergic mouse model against OVM, OVA, or casein. The mice 10-14 days after transdermal sensitization were subjected to the following experiments.

Hereinafter, each mouse model is sometimes referred to as "OVM allergic mouse of the present invention", "OVA allergic mouse of the present invention", and "casein allergic mouse of the present invention", respectively. These three types of mice are sometimes collectively referred to as "allergic mouse of the present invention".

### Example 2

### [Anaphylaxis-eliciting test using aspirin]

In food-allergic patients, allergic reactions including anaphylaxis are elicited by taking an allergen ingested orally into the body via intestinal mucosa. However, conventionally, as methods for causing development of anaphylaxis in a sensitized mouse model, intravenous or intraperitoneal injections of small amounts of allergens (dozens of µg to 1 mg/mouse) were generally used. Although these administration methods can reliably develop severe anaphylaxis, it is difficult to say that they accurately reflect the pathogenesis of food allergies in humans because the allergen uptake route is different from the oral route. Attempts have also been made to elicit anaphylaxis by orally administering large amounts of allergens (50-100 mg/mouse) to a mouse model, but it is known that there are large variations in degrees of anaphylaxis elicitation despite large amounts of oral allergen administration, and quantitative evaluation of the anaphylaxis suppression effect is difficult. From these, it has been desired to develop an oral allergen challenge method that elicits anaphylaxis in mouse model with high probability.

Meanwhile, aspirin (acetylsalicylic acid; ASA) is known as a substance that enhances symptoms of food allergies, and is also used in a food-dependent exercise-elicited anaphylaxis test in humans. Specifically, in a food-dependent exercise-elicited anaphylaxis diagnosis, aspirin has been sometimes used as an eliciting promoter in a method of diagnosing by combination with three kinds of loadings: "pre-administration of aspirin" and "food + exercise" (Brockow K et al. J Allergy Clin Immunol. 2015; 135: 977-984.e4).

In accordance with the schedule shown in Figure 1, the present inventors performed two types of loadings of "ASA pre-administration" and "allergen oral administration" in combination on the allergen transdermally sensitized mouse model of the present invention to examine whether anaphylaxis could be reliably elicited. Experimental procedures and results are shown in the following (1) to (4).

### (1) ASA pre-administration

The OVA transdermally sensitized model mice (body weight of about 20 g) of the present invention were fasted for 2 hours or more and then oral administration of ASA (manufactured by Sigma-Aldrich) dissolved in 50% ethanol (1.25 mg/100 µL/mouse) was performed. Also, as a control, a group was provided to which oral pre-administration of 50% ethanol alone without ASA was performed.

### (2) OVA oral challenge

Thirty minutes after the pre-administration of ASA or ethanol, an aqueous OVA solution (manufactured by Sigma-Aldrich) was orally challenged (20 mg/100 µL/mouse). In addition, as a control, a group was provided to which only water (without OVA) was orally administered.

### (3) Rectal temperature monitoring

Rectal temperature is commonly used as an indicator of anaphylaxis development and it is known that the lower rectal temperature shows the more severe symptoms of anaphylaxis. In this experiment, rectal temperatures were measured at 10-minute intervals starting from 10 minutes before OVA administration and for 90 to 120 minutes after administration. Then, the temperature change after OVA administration was monitored using the temperature 10 minutes before OVA administration as a reference value. It was determined that the mouse developed anaphylaxis when the rectal temperature decreased by 1°C or more relative to the reference value.

### (4) Results

The results are shown in Figure 2. Table 1 below shows which administration group corresponds to the notation in Figure 2.

**[Table 1]**

| Notations in Figure 2 | Pre-administration | Allergen challenge |
|---|---|---|
| ASA/50% ethanol + OVA | Administration of ASA (Solvent is ethanol) | OVA challenge (Solvent is water) |
| 50% Ethanol + OVA | Administration of only 50% ethanol | OVA challenge (Solvent is water) |
| ASA/50% ethanol | Administration of ASA (Solvent is 50% ethanol) | Administration of only water |
| OVA alone | None (Neither ASA nor ethanol is administered) | OVA challenge (Solvent is water) |

After pre-administration of ASA, in the group where OVA was orally administered ("ASA/50% ethanol + OVA " in Figure 2), a rapid decrease in rectal temperature occurred 30 minutes after the challenge of OVA (a decrease of about 2.8°C), and a decrease of 1.3°C was observed even after 90 minutes. This result is a clear indication that anaphylaxis was elicited in the administration group and that the symptoms did not recover even after 90 minutes.

In contrast, in the group where OVA was orally administered without pre-administration of ASA ("OVA alone" in Figure 2), rectal temperature decreased slightly (around 1.1°C) 10 minutes after the OVA challenge, and recovered in a short time, thus it was determined that elicitation of anaphylaxis was extremely mild.

In addition, in the group where only pre-administration of ASA was carried out, and OVA was not challenged ("ASA/50% ethanol" in Figure 2), a tendency of increase in rectal temperature was observed from after 30 minutes. Similarly, even in the group where 50% ethanol was pre-administered and OVA was orally challenged ("50% ethanol + OVA" in Figure 2), an increase in rectal temperature was observed from after 30 minutes. The increase in rectal temperature in these groups is presumed due to the effect of 50% ethanol administration and is believed to be unrelated to allergic symptoms. Also, in the "ASA/50% ethanol" group, an increase in rectal temperature was observed with a similar change over time.

From the above results, it was revealed that oral administration of ASA to the allergic mouse of the present invention beforehand promotes the elicitation of anaphylaxis caused by oral administration of an allergen.

### Example 3

### [Induction of HAv-specific IgA of systemic mucosa by nasal inoculation of complex of influenza antigen (HAv) with SF10]

As described above, complexes of influenza antigen (hemagglutinin; HAv) with SF10 (HAv-SF10) are already known to be useful as vaccines for nasal inoculation (Kimoto T, et al. Influenza and Other Resp. Viruses 7(6) :1218-1226, 2013.; Mizuno D, et al. Vaccine 34(16): 1881-1888, 2016.; Kim H, et al. PLOS ONE 13(1) :e0191133, 2018.). These articles have also revealed that nasal inoculation of HAv-SF10 induces HAv-specific IgA not only in blood, but also in nasal and bronchoalveolar lavage fluids.

The present inventors have then examined the effect of nasal inoculation of HAv-SF10 vaccine on induction of IgA production of systemic mucosa including the gastrointestinal tract. Specifically, mice (body weight of about 20 g) were nasally inoculated with HAv-SF10, and two weeks later, bronchoalveolar lavage fluids, nasal lavage fluids, vaginal fluids, and intestinal fluids (small intestinal fluids, large intestinal fluids (stool), small intestinal fluids + large intestinal fluids) were collected to measure the amount of HAv-specific IgA contained in each. Also, for comparison with SF10, Poly (I : C), a potent mucosal adjuvant commonly used in animal experiments of nasal inoculation vaccine (Ichinohe T, et al. J Virol. 2005; 79: 2910-2919.), was used to carry out a similar experiment.

The results are shown in Figure 3. It was revealed that, after nasal inoculation of HAv-SF10, the amount of HAv-specific IgA increases not only in nasal and bronchial lavage fluids, but also in vaginal and gastrointestinal secretions. In the saline and HAv alone administration groups, the amounts of HAv-specific IgA antibody were below the detection limit value, as shown in bronchial lavage fluids. Since this tendency was same in the nasal lavage fluids, the vaginal fluids, and the intestinal fluids, these indications are omitted. From the above, it was found that nasal inoculation of a complex of HAv with SF10 greatly increases the amount of HAv-specific IgA production compared to nasal inoculation of HAv alone. Furthermore, nasal inoculation of HAv-SF10 vaccine had the greatest effect on IgA production in the intestine, showing that the effect is equivalent to or higher than that of nasal inoculation vaccine containing Poly (I : C), suggesting that the adjuvant effect of SF10 is easy to appear in the gastrointestinal tract.

### Example 4

### [Effect of oral inoculation of food allergen-SF10 complex on promotion of uptake of food allergen into gastrointestinal mucosal dendritic cells]

From the results of Example 3, it was strongly suggested that SF10 adjuvant could affect antibody production in the gastrointestinal tract. Studies using rabbits have also reported that, when administered into amniotic fluid, a complex of human pulmonary surfactant with fetal fat is selectively absorbed into the gastrointestinal mucosa of rabbit fetus and promotes development of the mucosal epithelium at the site of absorption (Nishijima K, et al. Am J Physiol Lung Cell Mol Physiol. 2012; 303: L208-L214.). From these data, the present inventors considered that SF10 could be used as an antigen carrier in a vaccine for oral inoculation other than nasal inoculation, and examined the uptake of OVA into small intestinal mucosal dendritic cells by orally inoculating mice with a complex of OVA with SF10 (hereinafter sometimes referred to as "OVA-SF10"). Experimental procedures and results are shown in the following (1) to (4).

### (1) Preparation of fluorescently labeled OVA-SF10

A fluorescently labeled complex of OVA with SF10 was prepared according to known techniques (Mizuno D, et al. Vaccine 29(33):5368-5678, 2011.; Kimoto T, et al. Influenza and Other Resp. Viruses 7(6):1218-1226, 2013.; Mizuno D, et al. Vaccine 34(16) : 1881-1888, 2016.; Kim H, et al. PLOS ONE 13(1):e0191133, 2018.). In order to clearly measure the uptake of fluorescent dye-labeled OVA into small intestinal dendritic cells, for the amount of OVA in OVA-SF10 for oral inoculation, a large amount of antigen (100 µg/mouse with body weight of about 20 g) compared to that in oral inoculation antibody-inducing experiments (normal antigen amount: 0.2 to 1 µg/mouse with body weight of about 20 g) was used.

Specifically, OVA (manufactured by Sigma-Aldrich) labeled with a fluorescent dye Alexa 647, and SSF created by known techniques described above were mixed, and the mixture was lyophilized to generate a complex of fluorescently labeled OVA with SSF (which may hereinafter be referred to as "OVA-SSF"). In the above mixing, the phospholipids : OVA in SSF was adjusted to be 10 : 1 (mass mixing ratio). Immediately before oral inoculation, 0.5 mL of CVP (Hiviswako 104, manufactured by FUJIFILM Wako Pure Chemical Corporation) adjusted to 1.0% with saline per lyophilized OVA-SSF (lyophilized, 5.5 mg) was added, and the mixture was uniformly dissolved, and an equal amount of 50 mM carbonate buffer (pH 9.7) was further added in order to avoid inactivation of antigen by gastric acid to finally generate a fluorescently labeled OVA-SF10 complex (OVA-SF10). As a result of this series of operations, 200 µL of the vaccine solution to be inoculated per mouse contains 100 µg of OVA, 0.5% CVP, and 25 mM carbonate buffer.

### (2) Oral inoculation into mouse

Mice were anesthetized by intraperitoneal injection of ketamine (62.6 mg/kg body weight) and xylazine (12.4 mg/kg body weight). For each mouse (body weight of about 20 g), the above OVA-SF10 solution (200 µL) was inoculated directly into the stomach of mice using a feeding needle. Also, as a control, a group was provided in which Alexa647-labeled OVA (100 µg/200 µL, 25 mM carbonate buffer) was similarly orally inoculated into each mouse.

### (3) Sample collection and analysis

Small intestine mucosae were collected from the above mice 12 and 24 hours after the oral inoculation. In addition, according to known techniques (Harusato A, et al. Methods Mol Biol. 2016; 1422: 171-180.), mucosal epithelial layer and lamina propria cells were isolated from the small intestinal mucosa in the presence of 2 mM EDTA and 1.5 mg/mL collagenase type IV.

### (4) Flow cytometry analysis

The obtained cells were stained with dendritic cell marker antibodies (anti-mouse MHC class II (I-A/I-E) antibodies, CD11b antibodies, CD11c antibodies, and CD103 antibodies, manufactured by BioLegend). The percentage of Alexa647-positive cells in various dendritic cell groups was calculated by flow cytometry analysis (BD FACSVerse flow cytometer; manufactured by BD Bioscience), and the number of OVA uptake cells was measured.

### (5) Results

Figures 4B-E show the result of histogram analysis of uptake of fluorescent dye-labeled OVA in the respective dendritic cell populations of MHC II⁺CD11b⁺ cells (MHC II⁺CD11b⁺ dendritic cells), MHC II⁺CD11b⁺CD11c⁺CD103⁺ cells (CD103⁺ dendritic cells), MHC II⁺CD11b⁺CD11c⁺CD103⁻ cells (CD103⁻ dendritic cells) contained in the small intestinal mucosal epithelial layer and lamina propria shown in Figure 4A. The samples corresponding to Figures 4B-E are shown in Table 2 below.

**[Table 2]**

| | Cell type | Time from administration to collection |
|---|---|---|
| Figure 4B | Small intestinal mucosal epithelial layer cells | 12 hours |
| Figure 4C | Small intestinal mucosal lamina propria cells | 12 hours |
| Figure 4D | Small intestinal mucosal epithelial layer cells | 24 hours |
| Figure 4E | Small intestinal mucosal lamina propria cells | 24 hours |

Figures 4F-H are the results of the uptake cell number of fluorescent dye-labeled OVA shown in a bar graph in each of three types of dendritic cells: one is MHC II⁺CD11b⁺ dendritic cells shown in F; and the MHC II⁺CD11b⁺ dendritic cells is further divided into the other two, CD11b⁺,CD103⁺ dendritic cells and CD11b⁺,CD103⁻ dendritic cells.

As shown in Figures 4F-H, 12 hours after oral vaccination, the OVA-SF10 vaccination group had a significantly higher number of fluorescent dye-labeled OVA uptake cells in all three types of the dendritic cells (MHC II⁺CD11b⁺ dendritic cells, CD11b⁺,CD103⁺ dendritic cells, and CD11b⁺,CD103⁻ dendritic cells) compared to OVA alone oral inoculation. Meanwhile, 24 hours after oral administration, the signal of OVA taken up into dendritic cells was attenuated to about the detection limit in any of the cells.

From the above results, it has been revealed that oral inoculation of antigen in combination with SF10 enables antigen to be efficiently taken up into dendritic cells in the small intestinal mucosa. Taking into consideration that Poly (I : C), CpG, and the like known to date as potent adjuvants directly stimulate dendritic cells to promote antibody production, the adjuvant action of SF10 confirmed in this experiment can be said to be unique to promote antigen delivery effect. It should be noted that since it has been revealed that adjuvant effects of lung surfactant, which is the main source of SF10 and SSF, does not exhibit a direct stimulatory effect on dendritic cells like Poly (I : C) or CpG, it can be said that SF10 is a highly safe adjuvant (Mizuno D, et al. J Immunol, 176: 1122-1130, 2006). Dendritic cells that recognize antigen information in the gastrointestinal tract are thought to subsequently migrate to systemic lymph nodes to allow systemic production of antigen-specific IgG and IgA.

### Example 5

### [Increased production of antigen-specific IgG and IgA in systemic lymph nodes after oral inoculation of HAv-SF10 vaccine]

In Example 3, nasal inoculation of HAv-SF10 was shown to promote HAv-specific IgA production in the gastrointestinal tract and other organs as well. Then, the present inventors examined how oral inoculation of HAv-SF10 vaccine affects antigen-specific IgG and IgA production of lymphocytes in systemic lymph nodes. Experimental procedures and results are shown in the following (1) to (4).

### (1) Oral inoculation of HAv-SF10 vaccine

HAv-SF10 containing 1 µg of HAv (phospholipids : HAv in SSF is 10 : 1 (mass mixing ratio)) was prepared, and orally inoculated into mice (body weight of about 20 g) (1 µg HAv/200 µL/mouse). It should be noted that no carbonate buffer was used in the preparation of HAv-SF10 vaccine, because HAv is relatively less susceptible to gastric acid. The HAv-SF10 vaccination was orally inoculated with the same amount of HAv-SF10, a total of 4 times of after 3 days, 14 days, and 17 days after the initial administration (booster immunization). As a control, a group was provided in which HA alone was orally inoculated in a similar schedule.

### (2) Collection and culture of lymphocytes

Fourteen days after the final immunization (4th inoculation of HAv-SF10), lymphocytes were collected from the lungs, spleen, cervical lymph nodes, mediastinal chest lymph nodes, Peyer's patch, and gastric lymph nodes, respectively. The obtained lymphocytes (1 x 10⁵ to 1 x 10⁶) were seeded onto plates coated with HAv antigen, and cultured for 3 days. The culture was performed using RPMI1640 culture solution containing 1 µg/mL R848 (manufactured by Novus Biologicals), 10 ng/mL rmIL-2 (manufactured by BioLegend), 10 mM HEPES buffer, 1 mM sodium pyruvate, 1% non-essential amino acid solution, 14.3 µM 2-mercaptoethanol, 10 µg/mL gentamycin, and 10% heat-inactivated fetal serum.

### (3) Immunostaining

Immunostaining of cultured lymphocytes was carried out using an HRP-labeled anti-IgG antibody or an HRP-labeled anti-IgA antibody (manufactured by Sigma-Aldrich), and ELISpot assay was carried out. Cell number measurements were performed using LUNA-II(TM) Automated Cell Counter (manufactured by Logos Biosystems).

### (4) Results

On the left side of Figures 5A-F, staining images (photographs of IgA and IgG production spots) of lymphocytes from each organ are shown (A: lungs, B: spleen, C: cervical lymph nodes, D: mediastinal chest lymph nodes, E: Peyer's patch, F: gastric lymph nodes). The graphs on the right side of Figures 5A-F show the number of IgA or IgG-producing cells (IgA: circle, IgG: square, filled symbols are the mean values for each) per 1 × 10⁶ lymphocytes (N.D.: not-detected, *P < 0.05, **P < 0.01).

In the HAv alone administration group, very few IgG production spots were detected in lymphocytes from the cervical lymph nodes and the spleen, but no spot was detected in lymphocytes from other organs. In contrast, in the HAv-SF10 oral inoculation group, numerous IgG and IgA production spots were detected in lymphocytes from all organs. From these results, it was revealed that oral inoculation of SF10 in combination with antigen effectively induces a systemic immune response and strongly promotes antigen-specific IgG and IgA production.

### Example 6

### [Effect of HAv-SF10 on splenic cell cytokine secretion]

To investigate the mechanism of antibody production promoting action by oral inoculation of HAv-SF10 as observed in Example 5, HAv-specific IgG and IgA production of each systemic tissue after oral inoculation of HAv-SF10 and changes in cytokine secretion of splenocytes were examined. Experimental procedures and results are shown in the following (1) to (4).

### (1) Vaccination

HAv alone or HAv-SF10 was inoculated nasally and orally into mice (body weight of about 20 g). As a comparative control, a subcutaneous inoculation group of HAv was provided. The administration schedule was similar to that in the oral inoculation of Example 5 (a total of 4 times on days 0, 3, 14, and 17). In addition, as a control, an untreated group was provided. Experiments were carried out at n = 6 for each group.

### (2) Splenic cell culture and cytokine measurement

Two weeks after the final immunization, splenocytes were collected from the mice in each administration group, and cultured in the presence or absence of 10 µg/mL HA for three days. After culture, concentrations of IL-2, IFN-γ, IL-4, IL-5, IL-17A, and IL-22 in the culture medium were measured with LEGENDplex(TM) (manufactured by BioLegend). Hereinafter, IL-2 and IFN-γ are sometimes referred to as "Th1 cytokines", IL-4 and IL-5 as "Th2 cytokines", and IL-17A and IL-22 as "Th17 cytokines", respectively.

### (3) Measurement of HAv-specific antibody production amounts

Blood, bronchoalveolar lavage fluids, nasal lavage fluids, and stomach extracts, and stool extracts were collected from the mice in each immune group of (1) described above, and the concentrations of HAv-specific IgG and IgA were measured by enzyme-linked immunosorbent assay (ELISA) according to the previous report (Mizuno D, et al. J Immunol, 176: 1122-1130, 2006).

### (4) Results

The results of (2) above are shown in Figure 6. In the prepared mouse splenocytes, increased secretions of Th1, Th2, Th17 cytokines under HAv-stimulated culture conditions were observed in all immune systems. Among these, in the case of HAv alone immunization, it was revealed that the production of Th2 and Th1 cytokines is strongly promoted in the subcutaneous administration group (s.c.) compared to the nasal inoculation group (i.n.) or the oral inoculation group (p.o.). Meanwhile, when HAv-SF10 vaccine was used, it was revealed that secretion of TH17 cytokine and IL-2 is strongly increased in the oral inoculation group compared to the nasal inoculation group. In particular, for IL-17A, the secretion amount in the HAv-SF10 vaccine oral inoculation group was 6.6-fold higher than that in the nasal inoculation group, revealing a significant secretion-promoting effect. IL-17A has been reported to be involved in mucosal IgA secretion (Jaffar Z, et al. Eur J Immunol. 2009; 39: 3307-3314.; Hirota K, et al. Nat Immunol. 2013; 14: 372-379.), and it was estimated that HAv-SF10 oral vaccination is a unique vaccine that induces increase of IL-17A secretion. Such increased IL-17A secretion is considered to be possibly involved in the particular high IgA production promoting action of HAv-SF10 oral vaccination. For oral inoculation of HAv-SF10 vaccine, high IL-2 (Th1 cytokine) production and moderate IL-5 (Th2 cytokine) production were also observed in addition to Th17. These results were estimated to appear in high systemic HAv-specific IgA and IgG induction as shown below.

The results of (3) above are shown in Figures 7A-E (A: blood, B: bronchoalveolar lavage fluids, C: nasal lavage fluids, D: stomach extracts, E: stool extracts). The vertical axis of the graph shows HAv-specific antibody concentration (white column: IgA, black column: IgG) in the sample (mean + SEM, *P < 0.05, **P < 0.01) . In addition, the horizontal axis of the graph shows different immunization groups of respective inoculation routes (s.c.: subcutaneous inoculation group, i.n.: nasal inoculation group, p.o.: oral inoculation group).

As can be seen from the data in Figure 7 "s.c.", in the group of subcutaneous inoculation (the conventional administration method of influenza vaccines in Japan) of HAv, only IgG production was induced, and IgA antibody production was almost not induced. In contrast, in the oral inoculation group of HAv-SF10 vaccine (p.o.), productions of antigen-specific IgG and IgA were strongly promoted in all specimens. In blood specimens, compared to the HAv subcutaneous administration group (s.c.), the HAv-SF10 vaccine oral inoculation group (p.o.) had about a 6.7-fold increase in IgG and about a 180-fold increase in IgA. It has been reported that IgA is secreted in mucosa, which serves as an entry route for pathogens, and shows high cross-immunity (Tamura SI, et al. Eur J Immunol. 1992; 22: 477-481.). Thus, the results shown by this Example that oral inoculation of HAv-SF10 vaccine promotes production of IgA antibodies suggest that HAv-SF10 vaccine has excellent characteristics from the viewpoint of biological defense.

In nasal inoculation of HAv-SF10 vaccine, it was revealed that production amounts of antigen-specific IgG and IgA resulted in about 6.7-fold higher production of IgG in blood (A), while, on the contrary, production of IgA is promoted 10 to 200-fold more strongly than IgG in mucosal secretions such as bronchoalveolar lavage fluids (B), nasal lavage fluids (C), and stomach extracts (D). When comparing the nasal inoculation group and the oral inoculation group of HAv-SF10 vaccine, oral inoculation showed stronger IgG and IgA antibody production-inducing effects in all specimens, with the greatest difference in bronchoalveolar lavage fluids, showing about 700-fold higher antibody production for IgA and about 200-fold higher antibody production for IgG compared to the nasal inoculation group. In blood (A), oral inoculation showed about 20-fold higher antibody production amounts for both IgG and IgA than nasal inoculation.

As described above, from the results of Examples 5 and 6, it was revealed that oral inoculation of HAv-SF10 vaccine strongly induces HAv-specific IgG and IgA production in various immune organs of the whole body. Strong induction of antigen-specific IgA production, particularly in systemic mucosal secretions, remains unclear in the mechanism of action, but suggests that a strong IL-17A secretion-promoting action of HAv-SF10 in splenocytes may be involved.

### Example 7

### [Immune tolerance induction by oral inoculation of therapeutic OVM-SF10 immune tolerance-inducing vaccine]

OVM is known to be the most allergenic component in chicken eggs. Thus, using OVM transdermally sensitized allergic mice, an immune tolerance-inducing effect by oral inoculation of therapeutic vaccine of a complex of OVM with SF10 (hereinafter "OVM-SF10") was examined with anaphylaxis suppression effect as an indicator.

### (1) Preparation of therapeutic OVM-SF10 oral immune tolerance-inducing vaccine

In accordance with the techniques described in (1) of Example 4, OVM-SF10 oral immune tolerance-inducing vaccines containing various concentrations of OVM (1 µg, 0.1 µg, 0.01 µg) to be inoculated per mouse (body weight of about 20 g) were generated (hereinafter referred to as "OVM (1 µg)-SF10", "OVM (0.1 µg)-SF10", and "OVM (0.01 µg)-SF10", respectively). It should be noted that, in the preparation of the therapeutic oral immune tolerance-inducing vaccine, the preparation method is the same as in Example 4, but it is characterized in that the amount of antigen is trace. Specifically, OVM (1 µg, 0.1 µg, or 0.01 µg) and SSF containing phospholipids in 10-fold amount of OVM (10 µg, 1 µg, or 0.1 µg) were mixed, and the mixture was lyophilized. Immediately before oral administration, 1.0% CVP/saline (100 µL) was added to the lyophilized powder to dissolve, and then an equal amount of 50 mM carbonate buffer (pH 9.7) was further added to generate an OVM-SF10 vaccine solution (200 µL) to be inoculated orally into one mouse. As a result of this series of operations, in 200 µL of the therapeutic oral vaccine solution to be inoculated per mouse, the oral immune tolerance-inducing vaccine that contains 0.01 to 1 µg of OVM, 0.5% CVP, and 25 mM carbonate buffer are generated.

### (2) Vaccination of therapeutic OVM-SF10 oral immune tolerance-inducing vaccine

Transdermally sensitized OVM allergic mice which were brought in the anaphylaxis-eliciting condition beforehand were fasted for 2 hours, and then orally inoculated with therapeutic OVM-SF10 immune tolerance-inducing vaccine (200 µL). The vaccination was performed by orally inoculating with the same amount of therapeutic OVM-SF10 immune tolerance-inducing vaccine on 3 days, 14 days, and 17 days after the initial administration (a total of 4 inoculations). As a control group, a no vaccine administration group (sensitization only, no oral vaccine) was also provided.

### (3) Oral challenge anaphylaxis-eliciting test by OVM

In accordance with the procedures of Example 2, fasting and ASA administration before the challenge test were carried out, and then an oral allergen challenge test was carried out. Specifically, 14 days after the final immunization (4th OVM-SF10 vaccine administration), mice were fasted, and ASA was pre-administered, then oral challenge with OVM was performed (10 mg OVM/200 µL/mouse), and the rectal temperature was monitored over 120 minutes. As a control, mice that were not transdermally sensitized (non-sensitized mice) were orally challenged with OVM in the same way, and the monitoring of the rectal temperature was performed.

### (4) Results

The obtained data were shown in Figure 8 as a boxplot. The notations in each administration group and Figure 8 are as shown in Table 3 below. The thick solid line in Figure 8 indicates the median of rectal temperature of the "sensitization only (no vaccine)" group, and the thick dotted line indicates the median of rectal temperature of the "sensitization + vaccine 0.01 µg" group, respectively.

**[Table 3]**

| Notations in Figure 8 | Mouse | Inoculation of OVM-SF10 oral immune tolerance-inducing vaccine | OVM challenge test |
|---|---|---|---|
| No sensitization | Without transdermal sensitization | No | Yes |
| Sensitization only (no oral vaccine) | OVM transdermally sensitized | No | Yes |
| Sensitization + oral vaccine 0.01 µg | OVM transdermally sensitized | OVM (0.01 µg)-SF10 oral inoculation | Yes |
| Sensitization + oral vaccine 0.1 µg | OVM transdermally sensitized | OVM (0.1 µg)-SF10 oral inoculation | Yes |
| Sensitization + oral vaccine 1 µg | OVM transdermally sensitized | OVM (1 µg)-SF10 oral inoculation | Yes |

As shown in Figure 8, it was confirmed that in the non-sensitized mouse group, no change in rectal temperature was observed after the OVM challenge and that anaphylaxis was not elicited. In contrast, in the "sensitization only (no vaccine)" group (thick solid line), a peak in the decrease in rectal temperature of more than 1.7°C was observed 30 minutes after the OVM challenge, indicating that anaphylaxis was induced. These results support that the OVM transdermally sensitized allergic mouse of the present invention can be used as a mouse model presenting immediate allergic symptoms to OVM.

Meanwhile, in the therapeutic oral immune tolerance-inducing vaccine administration group, no decrease in rectal temperature after OVM challenge was observed in the "sensitization + oral vaccine 0.01 µg" group and it was confirmed that anaphylaxis development was almost completely suppressed. In addition, a slight decrease in rectal temperature after OVM challenge was observed in the "sensitization + oral vaccine 0.1 µg" and "sensitization + oral vaccine 1 µg" groups, indicating a slightly inferior tendency in the immune tolerance-inducing effect compared to the "sensitization + oral vaccine 0.01 µg" group.

From the above, it is considered that oral inoculation of therapeutic OVM-SF10 immune tolerance-inducing vaccine to an OVM transdermally sensitized allergic mouse induces immune tolerance. It was also shown that the action of the therapeutic OVM-SF10 oral immune tolerance-inducing vaccine exhibits a more potent immune tolerance-inducing effect when the OVM content is a trace amount.

### Example 8

### [Investigation of the optimal OVM content in therapeutic OVM-SF10 oral immune tolerance-inducing vaccine]

From the results of Example 7, it was suggested that the therapeutic OVM-SF10 immune tolerance-inducing vaccine effect (immune tolerance-inducing action) differed in immune tolerance-inducing effect depending on its OVM content in the vaccine. Thus, in Example 8, the optimal OVM content in therapeutic OVM-SF10 oral immune tolerance-inducing vaccine was investigated.

In accordance with the procedures of Example 7, therapeutic OVM-SF10 immune tolerance-inducing vaccines containing 0.001 µg to 1 µg of OVM were prepared (hereinafter referred to as "OVM (1 µg)-SF10", "OVM (0.1 µg)-SF10", "OVM (0.01 µg)-SF10", and "OVM (0.001 µg)-SF10", respectively). They were then orally inoculated into transdermally sensitized OVM allergic mice (body weight of about 20 g), and 14 days later, oral challenge test with OVM was carried out. Before the challenge test, fasting and ASA administration to the mice were performed according to Example 2.

A boxplot graph of the change in rectal temperature about 30 minutes after OVM challenge administration is shown in Figure 9. The mean value and median of rectal temperature were highest in the OVM (0.01 µg)-SF10 oral immune tolerance-inducing vaccine administration group and an anaphylaxis suppression effect was observed, indicating that the optimal content of OVM in the vaccine is 0.01 µg. Taking into consideration that the amount of OVM used for the conventional oral challenge test is 10 mg/mouse, it has been revealed that immune tolerance can be induced in the presence of SF10 with an extremely small amount of antigen (1/10,000,000 of the antigen (OVM)) used for challenge test.

### Example 9

### [Anaphylaxis-eliciting test with trace amount of OVM]

Examples 7 and 8 revealed that immune tolerance to OVM was established in mouse which had orally inoculation of an oral immune tolerance-inducing vaccine in which 0.01 µg OVM was combined with SF10. Then, in Example 9, it was investigated whether anaphylaxis could be induced when 0.01 µg of OVM that induced oral immune tolerance is orally inoculated alone in the absence of SF10 adjuvant. That is, it is an investigation of whether there is a risk that OVM released from the complex of OVM with SF10 would induce anaphylaxis.

Specifically, the transdermally sensitized OVM allergic mouse model (body weight of about 20 g, n = 10) was subjected to fasting and ASA administration before the challenge test in accordance with the procedures of Example 2. Then an oral OVM challenge test was performed. The oral OVM challenge test was performed by orally challenging with 0.01 µg of OVM once and examining change in rectal temperature for 60 minutes. As shown in the boxplot graph of Figure 10, the rectal temperature after 0.01 µg of OVM administration did not decrease in all mice tested. From this, it was revealed that oral administration of a trace amount 0.01 µg of OVM does not induce anaphylaxis even in transdermally sensitized OVM allergic mice.

Considering together the results of Examples 7-9, it was shown that even in mice that are in conditions ready for anaphylaxis elicitation, immune tolerance can be established by oral administration of a trace amount of OVM combined with SF10 adjuvant, that does not induce an immune response (anaphylaxis) by the allergen itself in the oral challenge of allergen alone. These results suggest that orally inoculating with this is useful as a therapeutic OVM-SF10 oral immune tolerance-inducing vaccine. In the case of a therapeutic oral vaccine composed of a complex of a trace amount of antigen with SF10, it has also been shown that the vaccine has a low risk of anaphylaxis even if the antigen is released from the complex, and the vaccine induces an effective immune tolerance. It is suggested that the OVM-SF10 oral immune tolerance-inducing vaccine has excellent activity as an agent for treating and preventing allergy.

### Example 10

### [Prevention of cow's milk allergy by oral inoculation of casein-SF10 complex immune tolerance-inducing vaccine]

Cow's milk allergies are difficult to induce immune tolerance, and many medical accidents due to anaphylaxis have been reported in the process of oral immunotherapy. The present inventors thus prepared a complex of casein with SF10 (hereinafter sometimes referred to as "casein-SF10") to examine the effect of the prophylactic casein-SF10 oral immune tolerance vaccine on suppression and prevention of anaphylaxis. It should be noted that, in the case of the prophylactic oral immune tolerance-inducing vaccine, it is believed that the vaccine is more effective when the amount of allergen used is larger than that in the therapeutic oral immune tolerance-inducing agent described in Example 7 because the vaccine is inoculated in the conditions where there is no allergy (conditions with no risk of developing anaphylaxis), and that the risk of adverse reactions is low even with a large amount of allergen.

Experimental procedures and results are shown in the following (1) to (4).

### (1) Preparation of prophylactic casein-SF10 oral immune tolerance-inducing vaccine

In accordance with the techniques of Example 4, a prophylactic casein-SF10 immune tolerance-inducing vaccine was prepared. Specifically, 10 mg of casein (manufactured by Sigma-Aldrich) and 100 mg of SSF created by known techniques (Kimoto T, et al. Influenza and Other Resp. Viruses 7(6):1218-1226, 2013.;Mizuno D, et al. Vaccine 34(16): 1881-1888, 2016.;Kim H, et al. PLOS ONE 13(1):e0191133, 2018.) were mixed, and the mixture was lyophilized to generate a complex of casein with SSF (casein-SSF). In the lyophilized mixture, phospholipids: casein in SSF was adjusted to be 10 : 1 (mass mixing ratio). Immediately before oral administration, 100 mL of 1.0% CVP (Hiviswako 104, manufactured by FUJIFILM Wako Pure Chemical Corporation) in saline was added to 1.1 mg of the lyophilized casein-SSF to uniformly dissolve, and then an equal amount of 50 mM carbonate buffer (pH 9.7) was added to adjust the final prophylactic oral immune tolerance-inducing vaccine solution (200 mL). By this series of operations, 200 µL of the prophylactic immune tolerance-inducing vaccine solution to be orally inoculated per mouse contains 1 µg of casein, 0.5% CVP, and 25 mM carbonate buffer.

### (2) Inoculation of prophylactic casein-SF10 immune tolerance-inducing agent

In accordance with the techniques of Example 4, 200 µL of the prophylactic casein-SF10 vaccine solution was orally inoculated into healthy mice (Balb/c mice, body weight of about 20 g), and three days later, the same amount of casein-SF10 vaccine was further orally inoculated (a total of two inoculations). One month after the final immunization, mice were subjected to the experiments in (3) below. As a control, a group of non-administration of casein-SF10 immune tolerance-inducing vaccine was also provided. Furthermore, to compare with the prophylactic casein-SF10 oral immune tolerance-inducing vaccine inoculation group about the prophylactic effect, a subcutaneous injection of casein alone (1 µg casein/100 µL saline; two times inoculation) group (a group of known subcutaneous immunotherapy) was provided. One month after the final immunization, the immunized mice were subjected to the experiments in (3) below.

### (3) Transdermal sensitization with casein

In accordance with the techniques of Example 1, transdermal sensitization with casein to the mice of (2) above was carried out. Specifically, an aqueous solution of casein (1 mg/100 µL) per time was applied to the back of the mice at five times a week for two weeks (a total of 10 times of application), thereby transdermally sensitization with casein was carried out. One month after the application sensitization, the following casein intraperitoneal challenge test was carried out. It should be noted that it is confirmed beforehand that under this transdermal sensitization condition with casein, the mice are brought in an anaphylaxis-eliciting conditions.

### (4) Anaphylaxis-eliciting test by intraperitoneal challenge with casein

To the mice in (3) above, casein was intraperitoneally administered (1 mg/mouse) and then rectal temperature was monitored for 100 minutes (n = 4 to 5). Elicitation of anaphylaxis by intraperitoneal or intravascular administration of an allergen expresses the most severe and intense immune response among anaphylaxis-eliciting tests. In the case of prophylactic vaccination, taking into consideration the potential for all allergen sensitization including transdermal allergen sensitization and intravascular sensitization, it is expected that the vaccine would also exhibit a prophylactic effect on the most severe anaphylaxis. From such backgrounds, the effects of inoculation of the prophylactic casein-SF10 immune tolerance-inducing vaccine were evaluated in a study of an inhibition of anaphylaxis-induction by intraperitoneal challenge with casein.

### (5) Results

The results are shown in Figures 11 and 12. The notations of each administration group in Figures 11 and 12 are as shown in Table 4 below.

**[Table 4]**

| Notations in Figures 11 and 12 | | Inoculation of prophylactic immune tolerance-inducing vaccine | Transdermal sensitization with casein | Intraperitoneal challenge test with casein |
|---|---|---|---|---|
| Figure 11(A) | "Mean of no transdermal sensitization, no vaccination" | No | No | Yes |
| | "Mean of transdermally sensitized, no vaccination" | No | Yes | Yes |
| | "Individual data of transdermally sensitized, no vaccination group" | No | Yes | Yes |
| Figure 11(B) | "Mean of no subcutaneous immunization, no transdermal sensitization" | No | No | Yes |
| | "Mean of transdermally sensitized, no subcutaneous immunization group" | No | Yes | Yes |
| | "Individual data of transdermally sensitized after subcutaneous immunization group" | Subcutaneous injection of casein | Yes | Yes |
| Figure 12 | "Mean of no oral immunization, no transdermal sensitization" | No | No | Yes |
| | "Mean of transdermally sensitized without oral immunization group" | No | Yes | Yes |
| | "Individual data of transdermally sensitized after oral immunization with casein-SF10 group" | Oral administration of casein-SF10 | Yes | Yes |

As shown in the "Mean of transdermally sensitized, no vaccination" group of Figures 11 and 12 (shown by thick dotted line), in the group in which only transdermal sensitization with casein was carried out without vaccine pre-administration (mean value of 5-4 mice), a significant decrease in rectal temperature of 2.8°C was observed with a peak of about 30-40 minutes after the intraperitoneal challenge with casein.

With respect to Figure 11(B), as shown in the "Individual data of transdermally sensitized after subcutaneous immunization group" (shown by thin solid line), in the group in which casein transdermal sensitization was carried out after pre-administration of casein subcutaneous injection (corresponding to subcutaneous immunotherapy), improvement of rectal temperature decrease after intraperitoneal challenge test was seen in only one case, and the other four cases rather revealed the worsening tendency of noticeable decrease in rectal temperature. These results show that conventional subcutaneous immunotherapy requires further investigation on its effect of preventing the development of milk allergy.

Meanwhile, as shown in Figure 12, in the group in which transdermal sensitization with casein was carried out after inoculation of the prophylactic casein-SF10 oral immune tolerance-inducing vaccine (shown by thin solid line), no noticeable decrease in rectal temperature was observed in all four mice despite performing an intraperitoneal challenge with casein. These results show that the inoculation of the prophylactic casein-SF10 oral immune tolerance-inducing vaccine is effective in preventing the development of casein allergy.

### Industrial Applicability

The immune tolerance-inducing agent of the present invention can be used for the curative treatment of allergic diseases that are recently increasing, as well as for the prevention of allergic diseases.

## Claims

1. An immune tolerance-inducing agent for oral administration, comprising a pulmonary surfactant-derived synthetic mucosal adjuvant SF10 and an antigen.

2. The immune tolerance-inducing agent according to claim 1, wherein the antigen is one or more antigens selected from ovomucoid, ovalbumin, and casein.

3. An agent for treatment or prevention of an allergic disease, comprising an immune tolerance-inducing agent for oral administration as an active ingredient, wherein the immune tolerance-inducing agent comprises a pulmonary surfactant-derived synthetic mucosal adjuvant SF10 and an antigen.

4. The agent for treatment or prevention of an allergic disease according to claim 3, wherein the treatment or prevention is an oral immunotherapy.

5. The agent for treatment or prevention of an allergic disease according to claim 3 or 4, wherein the allergic disease is a food allergy.

6. The agent for treatment or prevention of an allergic disease according to claim 5, wherein the food allergy is a cow's milk allergy or an egg allergy.
